(19) Europäisches Patentamt

European Patent Office

Office européen des brevets

(11) **EP 3 823 515 B1**

(12) **FASCICULE DE BREVET EUROPEEN**

(45) Date de publication et mention de la délivrance du brevet:
**28.02.2024   Bulletin 2024/09**

(21) Numéro de dépôt: **19752734.4**

(22) Date de dépôt: **16.07.2019**

(51) Classification Internationale des Brevets (IPC):
***A61B 3/024*** *(2006.01)*     ***A61B 5/16*** *(2006.01)*
***A61B 3/11*** *(2006.01)*

(52) Classification Coopérative des Brevets (CPC):
**A61B 3/024; A61B 3/112**

(86) Numéro de dépôt international:
**PCT/FR2019/051778**

(87) Numéro de publication internationale:
**WO 2020/016517 (23.01.2020 Gazette 2020/04)**

(54) **DISPOSITIF D'OBSERVATION OCULAIRE**

AUGENBEOBACHTUNGSVORRICHTUNG

OCULAR OBSERVATION DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **20.07.2018   FR 1856737**

(43) Date de publication de la demande:
**26.05.2021   Bulletin 2021/21**

(73) Titulaires:
• **SORBONNE UNIVERSITE**
  **75006 Paris (FR)**
• **Centre National de la Recherche Scientifique (CNRS)**
  **75016 Paris (FR)**
• **INSERM (Institut National de la Santé et de la Recherche Médicale)**
  **75013 Paris (FR)**

(72) Inventeurs:
• **LORENCEAU, Jean**
  **75013 Paris (FR)**
• **AJASSE, Suzon**
  **93110 Rosny sous Bois (FR)**

(74) Mandataire: **Plasseraud IP**
  **66, rue de la Chaussée d'Antin**
  **75440 Paris Cedex 09 (FR)**

(56) Documents cités:
WO-A1-92/12667      US-A1- 2010 085 539
US-A1- 2011 292 342

## Description

La présente invention concerne les techniques d'observation de l'oeil de personnes, et plus particulièrement les techniques visant à détecter des pathologies sur la rétine.

## ARRIERE-PLAN

Pour obtenir des informations sur l'état de la rétine d'une personne, on a recours soit à des observations de fond d'oeil, où la rétine est éclairée à travers la pupille et la lumière réfléchie est recueillie par un appareil tel qu'un ophtalmoscope, soit à des tests subjectifs, comme ceux basés sur la grille d'Amsler ou le champ visuel de Humphrey.

Si ces méthodes permettent de détecter des scotomes, voire de les localiser, elles ne sont pas dépourvues d'inconvénients. Le fond d'oeil prend un certain temps, nécessite généralement de dilater la pupille de la personne et peut lui causer de l'inconfort. Quant aux tests subjectifs, ils reposent sur le jugement de la personne et, de ce fait, peuvent être biaisés. Si on utilise un test subjectif pour localiser un scotome sur la rétine, il faut demander à la personne de détecter des cibles (points lumineux). Tester l'ensemble de la rétine peut prendre beaucoup de temps, typiquement plus de 10 à 20 minutes.

Il existe donc un besoin pour une technique simple, rapide et objective pour obtenir des informations sur d'éventuels scotomes affectant une personne.

L'invention vient répondre à ce besoin.

## RESUME

Il est ainsi proposé un dispositif d'observation oculaire, comprenant :

un détecteur produisant un signal dépendant de variations du diamètre pupillaire d'un oeil d'un sujet ;

un afficheur pour présenter au sujet un motif lumineux subdivisé en plusieurs régions, l'afficheur étant commandé pour que chaque région ait une intensité lumineuse modulée à une fréquence de modulation respective, les fréquences de modulation étant différentes d'une région à l'autre, les fréquences de modulation sont choisies de sorte qu'aucune d'elles ne soit une harmonique d'une autre; et

un analyseur recevant le signal du détecteur et analysant son contenu fréquentiel.

Le dispositif est utilisable pour mettre en oeuvre un procédé d'observation oculaire visant à tester la rétine d'un sujet. Ce procédé comprend :

présenter au sujet un motif lumineux subdivisé en plusieurs régions, chaque région ayant une intensité lumineuse modulée à une fréquence de modulation respective, les fréquences de modulation étant différentes d'une région à l'autre ;

détecter des variations de diamètre pupillaire d'un oeil du sujet ;

transformer les variations de diamètre pupillaire dans un domaine fréquentiel, pour obtenir un signal transformé ; et

analyser le signal transformé aux fréquences de modulation.

Le test repose sur l'induction d'activés pupillaires périodiques provoquées par des modulations, par exemple sinusoïdales, de luminance réparties dans le champ visuel. La tâche du sujet est simplement de regarder le centre de l'afficheur pendant une durée relativement courte. Si la réponse enregistrée par le détecteur et analysée sur une fréquence associée à l'une des régions présente un niveau relativement bas, c'est un indice que le sujet présente vraisemblablement un scotome dans une région correspondante de la rétine.

Dans une réalisation, les régions du motif lumineux sont en nombre inférieur ou égal à 10.

Au cours de l'analyse du contenu fréquentiel du signal du détecteur par l'analyseur, il est possible de discriminer efficacement les réponses pupillaires induites respectivement par la modulation d'intensité lumineuse de chaque région du motif lumineux présenté au sujet.

Dans une réalisation, les fréquences de modulation sont comprises entre 0,1 et 10 Hz.

Dans une réalisation, les fréquences de modulation sont comprises entre 0,5 et 5 Hz.

Le test peut être relativement rapide. Ainsi, l'afficheur peut être commandé pour présenter le motif lumineux au sujet et l'analyseur peut analyser le signal du détecteur pendant une durée inférieure à deux minutes.

Dans une réalisation, les régions du motif lumineux comprennent une première région située au centre du motif et des secondes régions occupant des secteurs angulaires autour de la première région. Les secondes régions du motif lumineux peuvent être réparties radialement en plusieurs couches autour de la première région. Dans le choix des fréquences de modulation affectées aux régions, on peut affecter la plus grande à la première région au centre du motif lumineux.

Dans une réalisation du dispositif, l'analyseur est configuré pour prendre en compte la puissance que présente une transformée du signal du détecteur dans le domaine fréquentiel aux fréquences de modulation. Il peut être configuré pour prendre en compte aussi la phase que présente la transformée du signal aux fréquences de modulation.

## BREVE DESCRIPTION DES DESSINS

D'autres particularités et avantages de la présente invention apparaîtront dans la description ci-après d'un exemple de réalisation non limitatif, en référence aux dessins annexés, dans lesquels :

- la figure 1 est un schéma synoptique d'un dispositif selon l'invention ;

- la figure 2 est un diagramme illustrant un exemple de motif lumineux utilisable par un dispositif selon l'invention ; et

- les figures 3 et 4 sont des graphiques illustrant des exemples d'analyse de Fourier des modulations du diamètre de la pupille dans un dispositif selon l'invention.

## DESCRIPTION DE MODES DE REALISATION

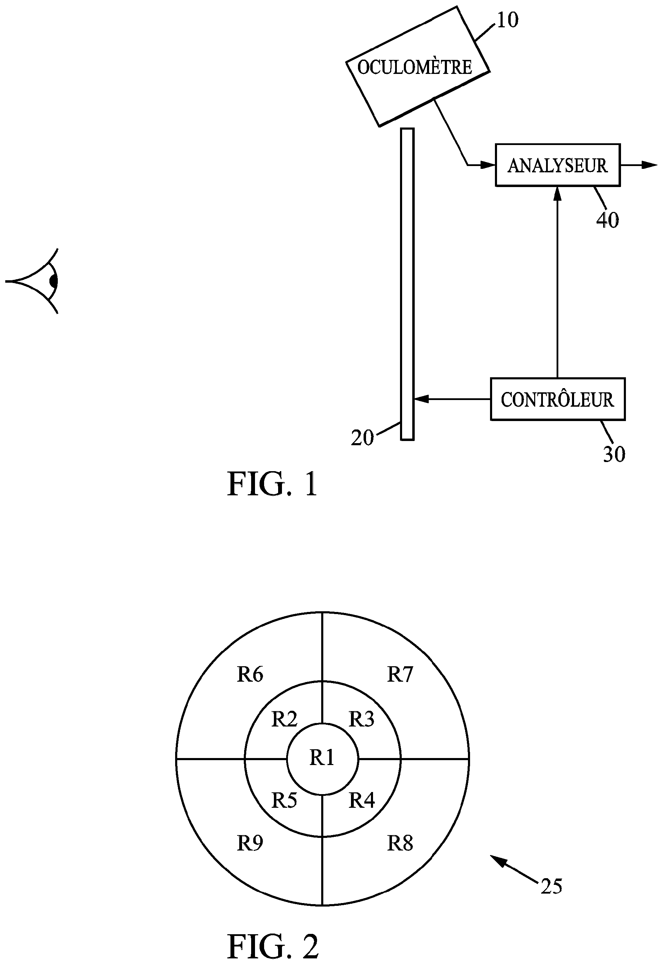

En référence à la figure 1, un mode de réalisation du dispositif selon l'invention comprend un détecteur 10, un afficheur 20 piloté par un contrôleur 30 et un analyseur 40.

Le détecteur 10 est capable de renseigner sur les variations de diamètre pupillaire d'un sujet placé en face de l'afficheur 20. Il peut notamment s'agir d'un oculomètre. L'oculomètre (« eye tracker » en anglais) est un appareil capable de mesurer un certain nombre de paramètres de l'oeil ou des yeux d'une personne, tels que la position de la pupille de l'oeil droit et/ou de l'oeil gauche, le diamètre pupillaire de l'oeil droit et/ou de l'oeil gauche, la direction du regard, la convergence et le synchronisme des activités des deux yeux. Dans l'application considérée ici, on pourra généralement se limiter aux informations sur le diamètre pupillaire, enregistrées au cours du temps.

Pour fiabiliser les mesures du détecteur 10, le dispositif peut encore comporter une mentonnière ou un autre système pour stabiliser la position de la tête du sujet en face de l'afficheur 20.

Les mesures sur un sujet par le dispositif selon l'invention sont typiquement effectuées oeil par oeil. Il est préférable de masquer l'oeil non mesuré.

L'afficheur 20 est par exemple un écran sur lequel est projeté un motif lumineux prédéfini. Cela peut aussi être un afficheur à diodes électroluminescentes ou à cristaux liquides. Dans un autre exemple, le détecteur 10 et l'afficheur 20 font partie d'un appareil du type pupillomètre.

Le contrôleur 30 pilote l'afficheur 20 pour que celui-ci présente au sujet un motif lumineux ayant plusieurs régions spatialement distribuées. Dans chaque région, la luminance du motif est modulée selon une caractéristique fréquentielle fixée par le contrôleur 20. Chaque région peut ainsi être associée à une fréquence de modulation respective. Le sujet est invité à placer son regard sur un point central du motif.

L'analyseur 40 reçoit le signal de sortie du détecteur 10 dépendant du diamètre pupillaire du sujet. Il analyse le contenu fréquentiel du signal dans la bande des fréquences de modulation employées dans le motif lumineux. Cette analyse utilise par exemple une transformée de Fourier des variations temporelles du diamètre pupillaire. D'autres transformées temps-fréquence sont, bien entendu, utilisables.

On attend a priori à ce que le spectre de Fourier ainsi calculé présente un pic à chaque fréquence de modulation employée dans le motif lumineux, car l'éclairement variable d'une portion de la rétine engendre normalement des fluctuations correspondantes du diamètre pupillaire. Si toutefois la rétine est atteinte d'un scotome, le pic sera affaibli dans le spectre de Fourier à la fréquence associée à la région du motif qui éclaire la zone scotomateuse.

Il est à noter que le diamètre pupillaire réagit au stimulus du motif lumineux par réflexe. Le test effectué à l'aide du dispositif ne requiert aucune tâche cognitive de la part du sujet. C'est un test objectif. On attend simplement du sujet qu'il fixe le centre du motif.

La durée du test peut être assez brève, typiquement de l'ordre de la minute ce qui permet au détecteur d'enregistrer un nombre d'oscillations de la luminance suffisant pour que le calcul de transformée de Fourier soit assez fin. Une durée inférieure à deux minutes a l'avantage de ne solliciter le sujet que peu de temps.

Dans le motif lumineux, la bande des fréquences de modulation est choisie selon les réponses physiologiques communément observées. La bande se trouve généralement dans une plage de 0,1 à 10 Hz, typiquement dans une plage de 0,5 à 5 Hz..

Un exemple de géométrie du motif lumineux affiché par l'afficheur 20 est représenté sur la figure 2. Ici, le motif a une région centrale R1 entourée par des régions périphériques R2-R9 réparties en deux couches selon la direction radiale. Les régions R2-R5 de la première couche radiale occupent quatre secteurs angulaires de 90° autour de la région centrale R1, tandis que les régions R6-R9 de la deuxième couche radiale occupent quatre secteurs angulaires de 90° autour des régions R2-R5.

Dans un cas particulier les fréquences de modulation de la luminance employées dans les régions R1 à R9 sont les suivantes :

- région R1 : modulation à 3,60 Hz ;
- région R2 : modulation à 3,25 Hz ;
- région R3 : modulation à 2,40 Hz ;
- région R4 : modulation à 1,25 Hz ;
- région R5 : modulation à 2,13 Hz ;
- région R6 : modulation à 1,75 Hz ;
- région R7 : modulation à 1,00 Hz ;
- région R8 : modulation à 2,75 Hz ;
- région R9 : modulation à 0,75 Hz.

On note que parmi ces neuf fréquences de modulation, la plus grande est celle associée à la région centrale R1 correspondant à la fovea. Il est nécessaire de choisir les fréquences en évitant, comme dans cet exemple, que l'une d'elles soit une harmonique d'une autre.

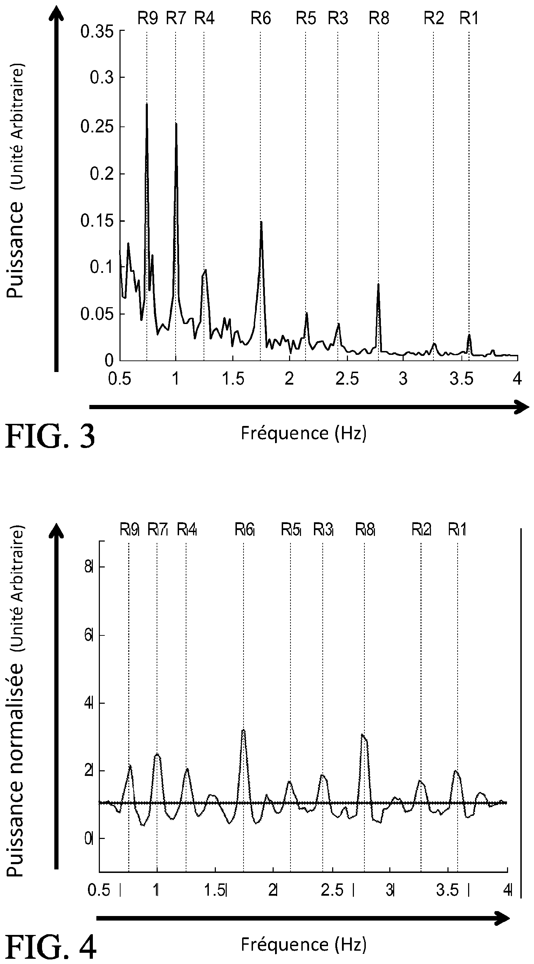

La figure 3 illustre des résultats obtenus sur l'oeil sain d'une personne, en montrant, en unité arbitraire, le module (puissance) $P_i$ de la transformée de Fourier discrète du signal du détecteur en fonction de la fréquence i, calculé par l'analyseur 40. Comme attendu, on y voit apparaître un pic à la fréquence de modulation de chacune des régions R1-R9. Dans l'exemple représenté, les fréquences de modulation sont telles qu'aucune d'elles ne soit une harmonique d'une autre. Ainsi, on évite le recouvrement entre un premier pic correspondant à la fréquence de modulation principale d'une première région et un deuxième pic correspondant à une fréquence de modulation harmonique d'une deuxième région. Ainsi, chaque pic permet de qualifier la réponse pupillaire du sujet à la modulation de la luminance d'une seule région concernée du motif lumineux.

De plus, le nombre de régions étant inférieur à 10, chaque pic est nettement séparé des pics adjacents sur le spectre représenté sur la figure 3. La réponse pupillaire du sujet à la modulation de la luminance de chaque région du motif lumineux peut ainsi être quantifiée, par exemple sur la base de la hauteur ou de l'intégrale des pics.

La figure 4 est une autre représentation des résultats obtenus sur le même oeil. Ici, la puissance est normalisée pour faciliter la visualisation et la comparaison des puissances aux différentes fréquences de modulation. La visualisation est plus facile avec ce format. La formule utilisée pour normaliser dans cet exemple particulier consiste à obtenir la puissance normalisée $P'_i$ à une fréquence i du spectre discret en divisant la puissance au voisinage de la fréquence i par celles des fréquences d'un voisinage élargi, selon :

$$P'_i = (P_{i-1} + P_i + P_{i+1}) / [(P_{i-4} + P_{i-3} + P_{i-2} + P_{i+2} + P_{i+3} + P_{i+4})/2]$$

D'autres méthodes de normalisation peuvent être utilisées, pourvu qu'elles répondent à l'objectif de normalisation permettant des comparaisons des puissances évoquées entre fréquences.

Si l'oeil d'un sujet est atteint d'un scotome, l'un au moins des pics de puissance visibles aux fréquences R1-R9 est diminué. La fréquence où la diminution est observée renseigne sur la localisation du scotome sur la rétine.

Des informations de phase, représentant le délai entre la variation de luminance et la réponse pupillaire peuvent être utilisées pour affiner les résultats obtenus en analysant la puissance $P_i$ ou $P'_i$.

La phase de la transformée de Fourier du signal du détecteur dépend du délai entre la stimulation et la réponse pupillaire, et constitue de ce fait une variable complémentaire permettant de détecter un scotome. On s'attend en effet à ce qu'une mauvaise transmission du signal rétinien se traduise par un déphasage plus important de la réponse. En associant aux régions R1-R9 des fréquences de modulation (stimulation) différenciées, on peut discriminer des régions répondant avec un déphasage anormal, indicatif d'un probable scotome. Une variable mixte, combinant puissance et déphasage aux fréquences de modulation, est donc judicieuse pour fournir une meilleure sensibilité pour la détection de zones déficitaires de la rétine.

Les avantages du test pupillo-fréquentiel effectué à l'aide du dispositif présenté ci-dessus incluent :

- absence d'une tâche subjective ;

- rapidité ;

- mesure objective liée à la mesure de la pupille ;

- capacité à localiser l'anomalie sur la rétine.

Ce test est applicable notamment au dépistage et au diagnostic de pathologies ophtalmologiques (dégénérescence maculaire, glaucome, etc.).

Les modes de réalisation décrits ci-dessus sont une simple illustration de la présente invention. Diverses modifications peuvent leur être apportées sans sortir du cadre qui ressort des revendications annexées.

**Revendications**

1. Dispositif d'observation oculaire, comprenant :

un détecteur (10) produisant un signal dépendant de variations du diamètre pupillaire d'un oeil d'un sujet ;
un afficheur (20) pour présenter au sujet un motif lumineux (25) subdivisé en plusieurs régions (R1-R9), l'afficheur étant commandé pour que chaque région ait une intensité lumineuse modulée à une fréquence de modulation respective, les fréquences de modulation étant différentes d'une région à l'autre ; et
un analyseur (40) recevant le signal du détecteur (10) et analysant son contenu fréquentiel,
le dispositif étant **caractérisé en ce que**
les fréquences de modulation sont choisies de sorte qu'aucune d'elles ne soit une harmonique d'une autre.

2. Dispositif selon la revendication 1, dans lequel les fréquences de modulation sont comprises entre 0,1 et 10 Hz.

3. Dispositif selon la revendication 2, dans lequel les fréquences de modulation sont comprises entre 0,5 et 5 Hz.

4. Dispositif selon l'une quelconque des revendications précédentes, dans lequel les régions (R1-R9) du motif lumineux sont en nombre inférieur ou égal à 10.

5. Dispositif selon l'une quelconque des revendications précédentes, dans lequel l'afficheur (20) est commandé pour présenter le motif lumineux (25) au sujet pendant une durée inférieure à deux minutes et l'analyseur (30) analyse le signal du détecteur (10) sur ladite durée.

6. Dispositif selon l'une quelconque des revendications précédentes, dans lequel les régions du motif lumineux (25) comprennent une première région (R1) située au centre du motif et des secondes régions (R2-R8) occupant des secteurs angulaires autour de la première région.

7. Dispositif selon la revendication 6, dans lequel les secondes régions (R2-R8) du motif lumineux (25) sont réparties radialement en plusieurs couches autour de la première région (R1).

8. Dispositif selon la revendication 6 ou la revendication 7, dans lequel la fréquence de modulation est plus grande dans la première région (R1) que dans les secondes régions (R2-R8) du motif lumineux (25).

9. Dispositif selon l'une quelconque des revendications précédentes, dans lequel l'analyseur (40) est configuré pour prendre en compte la puissance que présente une transformée du signal du détecteur (10) dans le domaine fréquentiel aux fréquences de modulation.

10. Dispositif selon la revendication 9, dans lequel l'analyseur (40) est configuré pour prendre en compte, en outre, la phase que présente la transformée du signal du détecteur (10) aux fréquences de modulation.

**Patentansprüche**

1. Augenbeobachtungsvorrichtung, umfassend:

einen Detektor (10), der ein Signal erzeugt, das von Veränderungen des Pupillendurchmessers eines Auges eines Subjekts abhängig ist;

eine Anzeige (20), um dem Subjekt ein Lichtmuster (25) zu präsentieren, das in mehrere Bereiche (R1-R9) unterteilt ist, wobei die Anzeige so gesteuert wird, dass jeder Bereich eine Lichtintensität aufweist, die mit einer jeweiligen Modulationsfrequenz moduliert ist, wobei sich die Modulationsfrequenzen von einem Bereich zum anderen unterscheiden; und

einen Analysator (40), der das Signal vom Detektor (10) empfängt und dessen Frequenzinhalt analysiert, wobei die Vorrichtung **dadurch gekennzeichnet ist, dass**

die Modulationsfrequenzen so gewählt werden, dass keine von ihnen eine Harmonische einer anderen ist.

2. Vorrichtung nach Anspruch 1, wobei die Modulationsfrequenzen im Bereich zwischen 0,1 und 10 Hz liegen.

3. Vorrichtung nach Anspruch 2, wobei die Modulationsfrequenzen im Bereich zwischen 0,5 und 5 Hz liegen.

4. Vorrichtung nach einem der vorstehenden Ansprüche, wobei die Bereiche (R1-R9) des Lichtmusters eine Anzahl von kleiner oder gleich 10 aufweisen.

5. Vorrichtung nach einem der vorstehenden Ansprüche, wobei die Anzeige (20) so gesteuert wird, dass sie dem Subjekt das Lichtmuster (25) während einer Dauer von kleiner als zwei Minuten präsentiert, und der Analysator (30) das Signal des Detektors (10) über diese Dauer analysiert.

6. Vorrichtung nach einem der vorstehenden Ansprüche, wobei die Bereiche des Lichtmusters (25) einen ersten Bereich (R1), der sich in der Mitte des Musters befindet, und zweite Bereiche (R2-R8) umfassen, die Winkelsektoren um den ersten Bereich herum einnehmen.

7. Vorrichtung nach Anspruch 6, wobei die zweiten Bereiche (R2-R8) des Lichtmusters (25) radial in mehreren Schichten um den ersten Bereich (R1) herum verteilt sind.

8. Vorrichtung nach Anspruch 6 oder Anspruch 7, wobei die Modulationsfrequenz im ersten Bereich (R1) höher ist als in den zweiten Bereichen (R2-R8) des Lichtmusters (25).

9. Vorrichtung nach einem der vorstehenden Ansprüche, wobei der Analysator (40) so konfiguriert ist, dass er die Leistung berücksichtigt, die eine Transformierte des Signals des Detektors (10) im Frequenzbereich bei den Modulationsfrequenzen aufweist.

10. Vorrichtung nach Anspruch 9, wobei der Analysator (40) so konfiguriert ist, dass er weiter die Phase berücksichtigt, die die Transformierte des Signals des Detektors (10) bei den Modulationsfrequenzen aufweist.

## Claims

1. An eye observation device, comprising:

a detector (10) that produces a signal dependent on variations in the pupil diameter of an eye of a subject;

a display (20) for presenting to the subject a luminous pattern (25) that is subdivided into a plurality of regions (R1-R9), the display being controlled so that each region has a light intensity modulated at a respective modulation frequency, the modulation frequencies being different from one region to the next; and

an analyzer (40) that receives the signal of the detector (10) and that analyzes its frequency content, the device being **characterized in that**

the modulation frequencies are chosen so that none of them is a harmonic of another.

2. The device as claimed in claim 1, wherein the modulation frequencies are comprised between 0.1 and 10 Hz.

3. The device as claimed in claim 2, wherein the modulation frequencies are comprised between 0.5 and 5 Hz.

4. The device as claimed in any one of the preceding claims, wherein the regions (R1-R9) of the luminous pattern are lower than or equal to 10 in number.

5. The device as claimed in any one of the preceding claims, wherein the display (20) is controlled so as to present the luminous pattern (25) to the subject for a time shorter than two minutes and the analyzer (30) analyzes the signal of the detector (10) for said time.

6. The device as claimed in any one of the preceding claims, wherein the regions of the luminous pattern (25) comprise a first region (R1) located in the center of the pattern and second regions (R2-R8) occupying angular sectors around the first region.

7. The device as claimed in claim 6, wherein the second regions (R2-R8) of the luminous pattern (25) are distributed radially in a plurality of layers around the first region (R1).

8. The device as claimed in claim 6 or claim 7, wherein the modulation frequency is higher in the first region (R1) than in the second regions (R2-R8) of the luminous pattern (25).

9. The device as claimed in any one of the preceding claims, wherein the analyzer (40) is configured to take into account the power that a transform of the signal of the detector (10) has in the frequency domain at the modulation frequencies.

10. The device as claimed in claim 9, wherein the analyzer (40) is configured to further take into account the phase that the transform of the signal of the detector (10) has at the modulation frequencies.

FIG. 1

FIG. 2

FIG. 3

FIG. 4